# EUROPEAN PATENT APPLICATION

(11) **EP 3 141 613 A1**
(43) Date of publication of application: **15.03.2017**
(21) Application number: 15184943.7
(22) Date of filing: 11.09.2015
(51) Int. Cl.: C12Q 1/68

(54) **DETECTION OF QUALITY OF SURFACE WATER**

(71) Applicant: Nederlandse Organisatie voor toegepast- natuurwetenschappelijk onderzoek TNO, 2595 DA 's-Gravenhage (NL)
(72) Inventor: SCHUREN, Frank Henri Johan, 2595 DA 's-Gravenhage (NL)
(74) Representative: V.O.

(57) **Abstract**

The present invention relates to a method for developing a classifier for classifying the trophic state of surface water comprising the steps of:
a. taking samples of surface water of different trophic states;
b. determining the nucleotide sequence of 18S RNA of micro-organisms in said sample;
c. selecting for those sequences that are unique for a specific trophic state. Further, the present invention relates to a method for classifying the trophic state of surface water comprising the steps of:
a. taking a surface water sample;
b. extracting the nucleotide sequence of the 18S RNA of the microorganisms in said sample;
c. checking for the presence of one or more of the nucleotide sequences depicted in any of Tables 1 - 6 in the extracted nucleotide sequences of step b;
d. classifying the sample on basis of the presence or absence of said sequences.

Further, a kit comprising an array for hybridization forms part of the invention.

## Description

The invention relates to the field of biological testing, more particularly the testing of biodiversity, especially the testing of the biodiversity in order to detect the quality of surface water.

Surface water makes up around 4% or 368,000 m2 of the European Union and its quality significantly impacts public health and commercial enterprises as well as flora and fauna. As this water is under continuous pressure from contamination by heavy metals and nutrient overload from fertilisers and pesticides and remainders of all kinds of chemical substances from human waste, it has to be regularly monitored to enable timely interventions.

For this purpose, surface water is generally divided into three main categories, dependent upon the level of nutrients that is provided in the water, also called the trophic state of the water. The three main classes are eutrophic, mesotrophic and oligotrophic. The classification of water into these categories is basically provided by Carlson's trophic state index (as e.g. used by the United States Environmental Agency for determining the trophic state of lakes). This index is defined as the total weight of algal biomass in a given water body. An oligotrophic lake is a lake with low nutrient content. These lakes have low algal production, and consequently, often have very clear waters, with high drinking-water quality. The bottom waters of such lakes typically have ample oxygen; thus, such lakes often support many fish species, like lake trout, which require cold, well-oxygenated waters. Ecologists use the term oligotrophic to distinguish unproductive lakes, characterized by nutrient deficiency, from productive, eutrophic lakes, with an ample or excessive nutrient supply. A eutrophic body of water, commonly a lake or pond, has high biological productivity. Due to excessive nutrients, especially nitrogen and phosphorus, these water bodies are able to support an abundance of aquatic plants. Usually the water body will be dominated either by aquatic plants or algae. When aquatic plants dominate the water tends to be clear. When algae dominate the water tends to be darker. The algae engage in photosynthesis which supplies oxygen to the fish and biota which inhabit these waters. Occasionally an excessive algae bloom will occur and can ultimately result in fish kills due to respiration by algae and bottom living bacteria.

Accordingly, the difference in the trophic state of surface water is not related to the (drinking) quality of the water, but more or less reflects the amount of water life that is present in the water.

However, changes in the trophic state may be indicator of ecological changes, not seldom caused by environmental changes, such as pollution. Accordingly, monitoring of the trophic state of surface water and changes therein is of utmost importance.

The current way of assessing the trophic state is cumbersome. An estimate of the state can be provided by measuring either the amount of chlorophyll pigments, the total phosphorus content or the so-called Secchi depth. Of these three the chlorophyll pigments will most accurately indicate the trophic state, but this changes with the season. The Secchi depth measurement is considered to be the least accurate, but is nowadays the cheapest and most readily available method. This parameter measures water transparency and by that would indicate the concentration of dissolved and particulate matter in the water, which in turn can be used to derive the biomass.

All of these parameters have the drawbacks that they are very general measurements, which may be influenced by other phenomena than the amount of nutrients (e.g. the season, sudden bursts of algae growth, and so on).

Currently the most reliable way to assay the trophic state of surface water is to take a sample and to look at the microbial lifeforms in the water, especially the diatom populations.

Diatomeae are unicellular algae of the phylum Bacillariophyta that are characterized by an exocellular silica skeleton. The wealth of species is enormous: it is estimated that there are more than 100,000 different species. They are water-bound and can be found in both deep and shallow waters, where they make up most of the phytoplankton. As such the population of diatoms in a water sample reflects the nutritional, i.e. trophic state of the water and thus diatoms can be used for the classification of the trophic state of surface water. However, currently this classification is performed by microscopic determination of the diatoms in the water sample and this method, although it is very reliable, is very labour intensive and thus costly.

Accordingly, an improved method for classification of the trophic state of surface water is needed.

### SUMMARY OF THE INVENTION

The present invention relates to a method for developing a classifier for classifying the trophic state of surface water comprising the steps of:
a. taking samples of surface water of different trophic states;
b. determining the nucleotide sequence of 18S RNA of micro-organisms in said sample;
c. selecting for those sequences that are unique for a specific trophic state.

Further, the present invention relates to a method for classifying the trophic state of surface water comprising the steps of:
a. taking a surface water sample;
b. extracting the nucleotide sequence of the 18S RNA of the microorganisms in said sample;
c. checking for the presence of one or more of the nucleotide sequences depicted in any of Tables 1 - 6 in the extracted nucleotide sequences of step b;
d. classifying the sample on basis of the presence or absence of said sequences.

Further, a kit comprising an array for hybridization forms part of the invention.

### DETAILED DESCRIPTION

The term "nucleic acid" as used herein, is interchangeable with the term "polynucleotide", and refers to a nucleotide multimer or polymeric form of nucleotides having any number of nucleotides, e.g., deoxyribonucleotides or ribonucleotides, or compounds produced synthetically (e.g. PNA as described in U.S. Pat. No. 5,948,902 and the references cited therein) and can be either double- or single-stranded. A polynucleotide can hybridize with other polynucleotides in a sequence specific manner, e.g. can participate in Watson-Crick base pairing interactions. The term also includes modified, for example by methylation and/or by capping, and unmodified forms of the polynucleotide

The terms "deoxyribonucleic acid" and "DNA" as used herein mean a polymer composed of deoxyribonucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming secondary structures (e.g., stem-loop, pseudo knots and kissing loop structures).

The term "nucleotide sequence homology" as used herein denotes the presence of homology between two polynucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by visual inspection. Readily available sequence comparison and multiple sequence alignment algorithms are, respectively, the Basic Local Alignment Search Tool (BLAST) (Altschul et al., 1990; Altschul et al., 1997) and ClustalW programs, both available on the internet. Other suitable programs include, but are not limited to, GAP, BestFit, PlotSimilarity, and FASTA in the Wisconsin Genetics Software Package (Genetics Computer Group (GCG), Madison, WI, USA) (Devereux et al., 1984). Especially the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482-489) should be mentioned.

As used herein, "substantially complementary" means that two nucleic acid sequences have at least about 70%, preferably about 80%, more preferably about 90%, even more preferably 95%, and most preferably about 98%, sequence complementarity to each other. This means that primers and probes must exhibit sufficient complementarity to their template and target nucleic acid, respectively, to hybridise under stringent conditions. Therefore, the primer and probe sequences need not reflect the exact complementary sequence of the binding region on the template and degenerate primers can be used. For example, a non-complementary nucleotide fragment may be attached to the 5'-end of the primer, with the remainder of the primer sequence being complementary to the strand. Alternatively, non-complementary bases or longer sequences can be interspersed into the primer, provided that the primer has sufficient complementarity with the sequence of one of the strands to be amplified to hybridize therewith, and to thereby form a duplex structure which can be extended by the polymerising means. The non-complementary nucleotide sequences of the primers may include restriction enzyme sites. Appending a restriction enzyme site to the end(s) of the target sequence would be particularly helpful for cloning of the target sequence. A substantially complementary primer sequence is one that has sufficient sequence complementarity to the amplification template to result in primer binding and second-strand synthesis. The skilled person is familiar with the requirements of primers to have sufficient sequence complementarity to the amplification template.

The term "hybrid" in the context of nucleic acids refers to a double-stranded nucleic acid molecule, or duplex, formed by hydrogen bonding between complementary nucleotide bases. The terms "hybridise" or "anneal" refer to the process by which single strands of nucleic acid sequences form double-helical segments through hydrogen bonding between complementary bases.

The term "probe" refers to a single-stranded oligonucleotide sequence that will recognize and form a hydrogen-bonded duplex with a complementary sequence in a target nucleic acid sequence or its cDNA derivative.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, i.e., in the presence of nucleotides and an agent for polymerisation such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerisation. The exact lengths of the primers will depend on many factors, including temperature and composition (A/T en G/C content) of primer. A pair of bi-directional primers consists of one forward and one reverse primer as commonly used in the art of DNA amplification such as in PCR amplification.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing.

Oligonucleotides, such as primers or the sequences as defined in any of SEQ ID NO: 1 - 97 may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in e.g. U.S. Pat. No. 4,458,066. The oligonucleotides may be labelled, if desired, by incorporating means detectable by for instance spectroscopic, fluorescence, photochemical, biochemical, immunochemical, or chemical means.

Template-dependent extension of the oligonucleotide primer(s) is catalysed by a polymerising agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyse primer- and template-dependent DNA synthesis. Known DNA polymerases include, for example, E. coli DNA polymerase I or its Klenow fragment, T4 DNA polymerase, and Taq DNA polymerase. The reaction conditions for catalysing DNA synthesis with these DNA polymerases are known in the art.

The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method is well described in handbooks and known to the skilled person.

After amplification by PCR, the (presence of) nucleic acids in a sample may be detected by hybridisation with a polynucleotide (designated as 'marker' or 'probe') which forms a stable hybrid with that of the target sequence under stringent to moderately stringent hybridisation and wash conditions. If it is expected that the probes will be essentially completely complementary (i.e., about 99% or greater) to the target sequence, stringent conditions will be used. If some mismatching is expected, for example if variant strains are expected with the result that the probe will not be completely complementary, the stringency of hybridisation may be lessened. However, conditions are chosen which rule out non-specific/adventitious binding. Conditions which affect hybridisation, and which select against non-specific binding are known in the art, and are described in, for example, Sambrook et al., (2001). Generally, lower salt concentration and higher temperature increase the stringency of binding. For example, it is usually considered that stringent conditions are incubations in solutions which contain approximately 0.1xSSC, 0.1% SDS, at about 65°C incubation/wash temperature, and moderately stringent conditions are incubations in solutions which contain approximately 1-2xSSC, 0.1% SDS and about 50°-65°C incubation/wash temperature. Low stringency conditions are 2xSSC and about 30°-50°C.

The terms "stringency" or "stringent hybridisation conditions" refer to hybridisation conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridisation with a buffer solution of 30% formamide, 1 M NaCl, 1% SDS at 37°C and a wash in 2x SSC at 40°C. Exemplary high stringency conditions include hybridisation in 50% formamide, 1 M NaCl, 1% SDS at 37°C, and a wash in 0.1x SSC at 60°C. Hybridisation procedures are well known in the art and are described by e.g. Ausubel et al., 1998 and Sambrook et al., 2001.

The term "hybridization pattern" refers to the list of measurements of spot intensities obtained after hybridizing the array with a sample comprising a variety of nucleic acids.

The term "nucleotide" is used to denote a deoxyribonucleoside, a ribonucleoside, or a 2'-O-substituted ribonucleoside residue.

The term "array" refers to an array of individual fragments of DNA or oligonucleotides that are bound to a substrate such as a microscope slide. The purpose of an array experiment is to determine the amount of matching nucleic acid fragments in a particular sample. The target nucleic acid fragments are extracted from the cells or tissues of interest, optionally converted to DNA, and labeled. They are then hybridized to all the DNA or oligonucleotide spots on the array. Matches (i.e. the spots where hybridization has taken place) are identified using antibody detection, optionally combined with precipitation, chromatography, colorimetry, and/or phosphorescent or fluorescent imaging. The data resulting from an array experiment are a list of measurements of spot intensities. A preferred array of the invention is an array which has less than 1000, preferably less than 500,more preferably less than 200, and preferably more than 50, more preferably more than 70, more preferably more than 80, more preferably more than 90, more preferably more than 95 spots, and which contains at least 50 of the DNA sequences of SEQ ID NO:1 - 97. Such an array may, next to the sequences that are actually designed for the detection or quantification of nucleotides in a sample, also contain sequences that are used as controls for the overall performance of the test.

For instance, arrays can be formed on non-porous surfaces (such as glass) by robotic micropipetting of nanoliter quantities of DNA to predetermined positions on a non- porous glass surface (as in Schena et aL, Science 270 : 467-470, 1995, and WO 95/35505). This is a "spotting" technique. Generally, in a spotting technique, the target molecules are delivered by directly depositing (rather than flowing) relatively small quantities of them in selected regions. For instance, a dispenser can move from address to address, depositing only as much target as necessary at each stop. Typical dispensers include an ink-jet printer or a micropipette to deliver the target in solution to the substrate, and a robotic system to control the position of the micropipette with respect to the substrate. In other embodiments, the dispenser includes a series of tubes, a manifold, an array of pipettes, or the like so that the target polypeptides can be delivered to the reaction regions simultaneously. In addition the oligonucleotide array can be synthesized by a light-directed chemical coupling, (see Pirrung et aL, (1992) U.S. Pat. No. 5,143, 854; Fodor et al, (1998) U.S. Pat. No. 5,800,992; Chee et al, (1998) U.S. Pat. No. 5,837,832. In brief, the light-directed combinatorial synthesis of oligonucleotide arrays on a glass surface proceeds using automated phosphoramidite chemistry and chip masking techniques. In one specific implementation, a glass surface is derivatized with a silane reagent containing a functional group, e.g., a hydroxyl or amine group blocked by a photolabile protecting group. Photolysis through a photolithogaphic mask is used selectively to expose functional groups which are then ready to react with incoming 5' photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences have been synthesized on the solid surface. Combinatorial synthesis of different oligonucleotide analogues at different locations on the array is determined by the pattern of illumination during synthesis and the order of addition of coupling reagents.

The data generated by assaying an array can be analyzed using known computerized systems. For instance, the array can be read by a computerized "reader" or scanner and quantification of the binding of probe to individual addresses on the array carried out using computer algorithms. Likewise, where a control probe has been used, computer algorithms can be used to normalize the hybridization signals in the different spots of the array. Such analyses of an array can be referred to as "automated detection" in that the data is being gathered by an automated reader system. In the case of labels that emit detectable electromagnetic wave or particles, the emitted light (e. g., fluorescence or luminescence) or radioactivity can be detected by very sensitive cameras, confocal scanners, image analysis devices, radioactive film or a Phosphoimager, which capture the signals (such as a color image) from the array. A computer with image analysis software detects this image, and analyzes the intensity of the signal for each probe location in the array. Signals can be compared between spots on a single array, or between arrays (such as a single array that is sequentially tested with multiple different samples), or between the labels of different probes on a single array. Computer algorithms can also be used for comparison between spots on a single array or on multiple arrays. In addition, the data from an array can be stored in a computer readable form. Certain examples of automated array readers (scanners) will be controlled by a computer and software programmed to direct the individual components of the reader (e. g., mechanical components such as motors, analysis components such as signal interpretation and background subtraction). Optionally software may also be provided to control a graphic user interface and one or more systems for sorting, categorizing, storing, analyzing, or otherwise processing the data output of the reader. To "read" an array, an array that has been assayed with a detectable molecule to produce binding (e. g., a binding pattern) can be placed into (or onto, or below, etc., depending on the location of the detector system) the reader and a detectable signal indicative of probe binding detected by the reader. Those addresses at which the molecule has bound to an immobilized nucleic acid probe provide a detectable signal, e. g., in the form of electromagnetic radiation. These detectable signals could be associated with an address identifier signal, identifying the site of the "positive" hybridized spot. The reader gathers information from each of the addresses, associates it with the address identifier signal, and recognizes addresses with a detectable signal as distinct from those not producing such a signal. Certain readers are also capable of detecting intermediate levels of signal, between no signal at all and a high signal, such that quantification of signals at individual addresses is enabled. Certain readers that can be used to collect data from the arrays, especially those that have been tested with using a fluorescently tagged molecule, will include a light source for optical radiation emission. The wavelength of the excitation light will usually be in the UV or visible range, but in some situations may be extended into the infra-red range. A beam splitter can direct the reader emitted excitation beam into the object lens, which for instance may be mounted such that it can move in the x, y and z directions in relation to the surface of the array substrate. The objective lens focuses the excitation light onto the array, and more particularly onto the (polypeptide) targets on the array. Light at longer wavelengths than the excitation light is emitted from addresses on the array that contain fluorescently-labeled probe molecules (i. e., those addresses containing a nucleic acid molecule within a spot containing a nucleic acid molecule to which the probe binds). In certain embodiments, the array may be movably disposed within the reader as it is being read, such that the array itself moves (for instance, rotates) while the reader detects information from each address. Alternatively, the array may be stationary within the reader while the reader detection system moves across or above or around the array to detect information from the addresses of the array (see 2001 WO 01/73134).

The term "trophic state" when relating to surface water, is herein defined as a measure for the indication of the level of nutrients that is present in the water. Although there is a classification into oligotrophic (little nutrients), mesotrophic (mediocre amount of nutrients) and eutrophic (high amount) and although there are even classes in between those (oligo-mesotrophic, meso-eutrophic) and even beyond those (hypereutrophic), it should be understood that classification is based on a sliding scale of an increase in the amount of nutrients. This scale does not recognize any decisive points where there is a clear distinction between two neighbouring classes. The borders of the classes will thus not be clearly defined and accordingly also classification of a sample into a class may be fuzzy.

The current invention provides a classification method for the trophic state of surface water.

It was found that it was possible to develop a set of biomarkers/probes that was able to distinguish between the different trophic states of surface water. This was achieved by taking samples of surface water with known characteristics (i.e. a confirmed trophic state), isolating the 18S nucleic acid from the micro-organisms in the sample and comparing the sequences found with sequences from other samples with the same trophic state. Doing this for the three trophic states (but also recognizing intermediate states like oligo-mesotrophic and meso-eutrophic) yielded a total of a few hundred sequences, some of which were uniquely found in a specific state, others that occurred in more than one state. From these many sequences now 97 sequences have been defined as being suitable for providing such a classification of surface water into one of the three, or preferably 5 or 6 trophic states.

The general method for finding these sequences can be described as a method for developing a classifier for classifying the trophic state of surface water comprising the steps of:
a. taking samples of surface water of different trophic states;
b. determining the nucleotide sequence of 18S RNA of micro-organisms in said sample;
c. selecting for those sequences that are unique for a specific trophic state. For such a method samples of water having a known trophic state need to be available.

To ensure that only the eukaryotic organisms are considered in the sample it has been chosen to look at the 18S RNA sequences, since these are characteristic for eukaryotic organisms. If needed, the water specimens first can be filtered to remove larger structures such as debris and larger eukaryotic organisms (seaweed, little molluscs and crustaceae). The 18S RNA is very suited for discovering the variation in a sample, because it contains highly conserved parts for which it is possible to prepare consensus primers for amplification using PCR or similar techniques. The amplicons that are produced in this way will next to the consensus parts also contain highly variable sequences that are specific on a family, genus, species or even variety level. It is this variety that enables the correlation the 18S sequences of the micro-organism population with the trophic state of the surface water.

The invention accordingly is also related to a method for the classification of the trophic state of surface water comprising the steps of:
a. taking a surface water sample;
b. extracting the nucleotide sequence of the 18S RNA of the microorganisms in said sample;
c. checking for the presence of one or more of the nucleotide sequences depicted in any of Tables 1 - 6 in the extracted nucleotide sequences of step b;
d. classifying the sample on basis of the presence or absence of said sequences.

As has been discussed above, extraction of the nucleotide sequence encoding the 18S rRNA subunit can be achieved by using an amplification of said nucleotide sequence with the use of consensus primers. In the current invention the use of the primers having the sequences **[5'-TGCAGTTAAAAAGCTCGTAG-3' and 5'-T**AATCATCTTCGATCCCCTA-3'**]** has been found very advantageous, since these primers anneal to a consensus sequence in the 18S rRNA coding sequence. Amplification with these primers should be performed under less stringent conditions to allow mismatches to be present.

After amplification the nucleic acids in the sample will be denaturised to make them single stranded. Thereafter will be applied to an array on which the probes (also indicated as biomarkers or marker sequences) have been provided. Amplicons that are complementary to the marker sequences will be bound to them and can be detected.

The sequences of markers given in the sequence listing are about 120 - 130 nucleotides long. However, this does not necessarily mean that the complete sequence as shown in any of the SEQ ID's needs to be present on the array: the assay can be been performed with sequences, which are parts thereof. It should, however, be understood, that a minimal length is necessary for obtaining a correct and distinctive hybridization with any sample nucleic acid. Thus, the length of these part(s) of the sequences should range from about 30 to about full length nucleotides, i.e. the sequences can be shorter han those presented in the sequence listing, but for a sufficient hybridization to occur they should be at least 30 nucleotides long and preferably contain the variable region which discriminates the 18S RNA sequence from other 18S RNA sequences. Especially the 5' and 3' ends may be truncated since these are consensus sequences that served as anchoring point for the primers.

Further, it will depend on the length of the probes and the hybridization conditions if and to what extent mismatches in the sequences would still lead to hybridization to the probe. Preferably, however, the hybridization assay is performed under stringent conditions to be certain that the probes do not a specifically bind to sequences in the sample thereby decreasing the reliability of the assay. Nevertheless, it is assumed that any nucleotide sequence or part thereof (as defined above) which has a homology of at least 90%, preferably at least 95%, more preferably at least 97%, even more preferably at least 98% with the sequences of SEQ ID NO: 1 through SEQ ID NO: 97 is applicable in and intended within the scope of the present invention.

In the current invention the term "homologues" is used to indicate both (parts of) the sequences of the invention of at least 30, but preferably at least 50, more preferably at least 75, more preferably at least 100 and most preferably at least about 120 nucleotides, and sequences which have a degree of homology of at least 90%, preferably at least 95%, more preferably at least 97%, even more preferably at least 98% with the sequences of SEQ ID NO: 1 through SEQ ID NO: 97.

The above discussed markers, or fragments thereof may be spotted on a surface to provide for a DNA micro-array. In order to facilitate coupling of the fragments, the surface of the array (e.g. the slide, the surface of which may i.a. be glass, gold, etc.) may be modified. Spotting may occur by any method available, for instance by using ElectroSpray Ionization (ESI) micro-array printing. After spotting of the markers or fragments thereof, the slide surfaces may be blocked to prevent further attachment of nucleic acids, e.g. by treatment with boro-anhydride in case of formaldehyde modified glass-slide surfaces.

To facilitate detection of successful hybridization, the sample DNA is suitably labeled, preferably with a compound which is uniquely detectable by an antibody. Such a compound can for instance be biotin. Labeling of nucleotides with biotin and subsequent detection through antibodies specific for biotin, has been sufficiently described in the literature and will be routine experimentation for a person skilled in the art. Alternatively, the nucleotides are fluorescently labeled (e.g. by using CyTM labels [Amersham Pharmacia Biotech]). Fluorescent labeling kits are commercially available from various manufacturers. In order to be able to judge the signals caused by the hybridization of the marker sequences with sample nucleic acid, preferably the array also comprises reference nucleotide sequences, which can serve as positive and negative controls. As negative controls, sequences should be used of about the same length as the average length of the markers, but which will not hybridize with any nucleotide sequence in the sample, i.e. sequences, which do not occur in eukaryotic 18S rRNA coding sequences As positive control, a sequence should be used which will be present in (nearly) all samples to be tested, for instance through adding a known amount of an artificial sequence to the sample just before testing.

The average size of sample nucleic acid, which is defined by the primers that are chosen for the amplification procedure, has an effect on the signal distribution on the array. Larger sample molecules comprise more information and are thus more likely to find a suitable hybridization partner in more of the spots. Reducing the average size of the sample nucleic acid can reduce this phenomenon. On the other hand, when the sample nucleic acid is too small, the nucleic acid fragments in the sample contain too little genetic information and also find suitable hybridization partners in many spots. The average size of the fragments in the sample nucleic acid is preferably between about 30 and 3000 nucleotides. More preferably, the average size of the fragments in the sample nucleic acid comprises a size of between about 50 and 1000 nucleotides, more preferably between about 100 and 500 nucleotides.

After performance of the hybridization assay the pattern that shows up from the (labeled) sequences that are detected shows the trophic class in which the sample can be classified. Below are six Tables over which in total 97 sequences (SEQ ID NO: 1- 97) are listed (in most cases there is overlap between consecutive Tables) showing which sequences would be typically found for each of the six trophic states. If a classification into three classes is performed than only Tables 1, 3 and 5 may be considered, although also in this case it would be advisable to additionally use a large part, if not all markers of Tables 2, 4 and 6. If an unknown sample is tested and all 97 sequences would be available on the array for hybridizing, it simply results in counting from which Table the most sequences are recognized; the sample then will be classified according to the class of the Table where the most hits are found. It has, however, been found that an acceptable classification may be performed with about 30 of the 97 sequences of Tables 1 - 6, which means that 5 sequences from every Table of the Tables 1 - 6 would be sufficient for a classification that still would be acceptable from a commercial perspective. Preferably however, because a larger amount of probes increases the performance of the assay, at least 40, more preferably at least 50, more preferably at least 70, more preferably at least 80, more preferably at least 90, more preferably at least 95 of the sequences of Table 1- 6 should be used in a hybridisation assay fort he classification of the trophic state of surface water. However, since the trophic state presumable has a Gaussian distribution over the classes, it would be advisable to take more samples from the middle classes (Tables 2-5) than from the extreme classes (Table 1 and 6).

It will, of course, be possible to add more sequences to the 97 sequences that are presented in the Table below. It is expected that performing a further search for additional sequences that can be used in the classification will provide further useful sequences, especially if samples are taken from climatologic different origin. The origin of the present set of probes is from a variety of surface water sources in the Netherlands , and thus would represent at least a variety that is useful for classification of a surface water sample derived from Northern-Europe. Although it is believed that the present set of markers would also be adequate for classifying surface water samples from the rest of the world, it is expected that when the current panel is completed with markers that were found using a test panel of surface water from other parts of the world and/or specific climatologic conditions, the performance of the classification can be improved.

Next to the *per se* classification of unknown samples, the present assay can also be advantageously used in monitoring the water quality by looking at changes over time in samples from the same origin. Although the trophic state of water may be subject to seasonal variation (see introduction) monitoring for detecting any positive or negative changes in the quality of the surface water. In such a case a full array as described above may be used, but it would also be possible to use just those markers that are predictive for the class in which the sample was originally classified (and maybe its neighbouring classes) to see if it would still fall within that class. For such applications arrays may be produced which consist of the markers of only one or a few classes.

Although not specifically designed for quantitative measurements, the assay of the present invention can be used to at least get an impression of the total amount of micro-organisms that are present in the sample. This can be derived from the intensity of the label at the spot(s) where a particular biomarker is present. If, for instance, the label is a fluorescent label, the emission of fluorescent radiation will be higher if more label (i.e. more target sequences from the sample) is hybridized to the marker sequence. Of course, this semi-quantitative measurement can be turned into a reliable quantitative measurement if there is a control measurement which has a known concentration and if the signal provided by the label is linear with respect to the concentration of the label at the spot. Further, it should be ensured that the spot is provided with sufficient marker molecules to enable binding to multiple target sequences from the sample.

Also part of the invention is a kit for the classification of the trophic state of surface water, wherein said kit comprises an array for hybridization optionally together with the necessary reagents for performing the hybridization assay, such as buffers, etc. , Said array is provided with at least 30 spots, each spot containing a sufficient number of molecules comprising one of the sequences as depicted in any of Tables 1 - 6. The nucleic acids may be bound to non-nucleic acid moieties for immobilization on the surface of the array plate as has been defined above. In one spot there should be sufficient identical molecules to enable differentiation in intensity when many identical target molecules are present in the sample to be tested. Next to the spots with sequences derived from the below Tables, the array may further contain positive and negative control sequences and optionally also additional areas for measurement of e.g. pH, oxygen concentration and the like.

**Table 1 Diatomeae 18S marker sequences for oligotrophic surface water**

| Class | SEQ ID NO | sequence |
|---|---|---|
| uniqueoligotroof_66 | 1 | TTGGATTTCTGGTTTGAGCGTCCGGTCGACCTTGTGTCTGTA CTATGGATGTTCGAGCCATATTTTGTGAGGATGCCTTTCTGC CATTGAGTTGGTGGTTGGGTGAACTTGCATCGTTTACTGTG |
| uniqueoligotroof_26 | 2 | TCGGATTTCGGGTGCTGCCCGGTGTAGCCAAGGCGAACGCC GGGTGGTGCCTTCCTTCTGGGATTCCTCTGCACTCTTCACTGA GTCGGATGGCGTTCCAGGACGTTTACTGTG |
| uniqueoligotroof_233 | 3 | TTGGATCTGGGGTTCGGGTCGGTGGTTTGCCGCTCTGCGGC TACTGCCGTGCTCGACTCTTACTAGCCGGTGCGTCTCTTGGT GCTCTTCATTGAGTGCCACGAGAGCCTGGCATGTTTACTTTG A |
| oligotroof_9 | 4 | TTGAATTTCTGGCAGGGGCCATCAAATTTCCACATGGTGGAT TGTGTGGTGGCCCCTGCCTTCTTTGGGTGAAGTTCCGGTGGC ATTAACTTGTTGCTGTTACGACATCCATCGTTTACTGTGA |
| oligotroof_10 | 5 | TTGGATTTGTGGTTGAGCTGTCCGGTCCGACACTTTGTGTG GGTACTTGGACGGGTCGTCCATCCTTGGGTGGAATCTGTGT GGCATTAGGTTGTCGTGCAGGGGATGCCCATCTTTTACTGTG |
| oligotroof_11 | 6 | TTGGATTTCTGGTCGCGTCCGCCGGTCCGGCCCTCACGGGTT GTGTACCTGGCGTGTGCAGCCATCCTCGGGGAGAGCACATC TGGGATTAAGTTGTCGGGTGTGGTATCCTCGTCATTTACTGT GA |
| oligotroof_13 | 7 | TTGAATTTCTGGCTCCGAATCGCGGTCTGGCCCAAGGGTTAT GTACCAGCGAGTTGGAGCCATCCTCGAGGAGAACTTATCTG GCATTCATTTGTTGGGTAAGAGACCCTCGTCATTTACTGTGA |
| oligotroof_14 | 8 | TTGAACCTTGGGCCTGGCTGGCCGGTCCGCCTCACCGCGTGC ACTGGTCCGGCCGGGTCTTTCCTTCTGAGGAGCCGCATGCCC TTCACTGGGTGTGTCGGGGAATCAGGACTTTTACTTTG |
| oligotroof_15 | 9 | TTGAATTTTGGACCTGGTTTAATGGTTAGGCCTTTGGCTTTTA CTATTGATTGGGTCTTTCCTTCTAGGGAACTTGTGTGCACTTT ATTGTGTGTGCAGGGGATCTAGGACTTTTACTTTG |
| oligotroof_20 | 10 | TTGAACCTTGGGCCTGGCTGGCCGGTCCGCCTCACCGCGTGC ACTGGCTCGGCCGGGTCTTTCCTCCTGGGGAGCCGCATGCCC TTCACTGGGTGTGTCGGGGAACCAGGACTTTTACTTTG |
| oligotroof_23 | 11 | TTGAATCTTGGGCCTGGTTTGGCGGTCGCGCTTTAGGGCGT GTACTGTCGGCCGGGTCTTTTACTTGCGGGGAACGTGCGTG CCTCTCGGGGTGTGTACGGGAACCGTGGCTATTACTTTG |
| oligotroof_37 | 12 | TTGGATTTCTGGTTTGAGCGTCCGGTCGAGTTTATCTCTGTAC TATGGATGTTTGAGCCATATTTGTGAGGATGCCTTTCTGCC ATTCAGTTGGTGGTTGGGTAGACTTGCATCGTTTACTGTG |
| oligotroof;oligo-mesotroof_48 | 13 | TTGGATTTGTGGCAGGTGCGTGCGGCCCGACACTGTGTGTG GGTGCTTGTGCGCGCCGCCATCCTTGGGTGGAACTTGTGTG GCATTAGGTTGTCGTGCAGGGGATGCCCATCTTTTACTGTG |
| oligotroof;oligo-mesotroof_53 | 14 | TTGGATTTCTGGTTGTGGACAGCTTTCAGCACTTCATTGTGTC TGACTTGTTGTCTGCGTCCATATTCGGGGGAAATTTATCTGG CATTAAGTTGTCGGGTAAAGGATCCTCGTCATTTACTGTGA |
| oligotroof;oligo-mesotroof_62 | 15 | TTGGATTTCTGCTGAGGACGATCGGTCCGCCCTCTGGGTGA GTATCTGACTCGGCCTTGGCATCTTCTTGGAGAACGTTACTG CACTTGACTGTGTGGTTCGGTATCCAGGACTTTTACTTTGA |
| oligotroof;oligo-mesotroof_64 | 16 | TTGAACCTTGGGCCTGGCTGGCCGGTCCGCCTCACCGCGTGC ACTGGTCCGGCCGGGCCTTTCCTTCTGGGGAGCCGCATGGC CTTCACTGGCCGTGTCGGGGAACCAGGACTTTTACTTTG |
| oligotroof;oligo-mesotroof_70 | 17 | TTGGATTTCTGGTTTGAGCGTCCGGTCGACCTTGTGTCTGTA CTATGGATGTTTGGGCCATATTTTGTGAGGATGCCTTTCTGC CATTGAGTTGGTGGTTGGGTAGACTTGCATCGTTTACTGTG |

**Table 2 Diatomeae 18S marker sequences for meso-oligotrophic surface water**

| Class | SEQ ID NO | sequence |
|---|---|---|
| oligotroof;oligo-mesotroof_48 | 13 | TTGGATTTGTGGCAGGTGCGTGCGGCCCGACACTGTGTGTGGGT GCTTGTGCGCGCCGCCATCCTTGGGTGGAACTTGTGTGGCATTAG GTTGTCGTGCAGGGGATGCCCATCTTTTACTGTG |
| oligotroof;oligo-mesotroof_53 | 14 | TTGGATTTCTGGTTGTGGACAGCTTTCAGCACTTCATTGTGTCTGA CTTGTTGTCTGCGTCCATATTCGGGGGAAATTTATCTGGCATTAAG TTGTCGGGTAAAGGATCCTCGTCATTTACTGTGA |
| oligotroof;oligo-mesotroof_62 | 15 | TTGGATTTCTGCTGAGGACGATCGGTCCGCCCTCTGGGTGAGTAT CTGACTCGGCCTTGGCATCTTCTTGGAGAACGTTACTGCACTTGAC TGTGTGGTTCGGTATCCAGGACTTTTACTTTGA |
| oligotroof;oligo-mesotroof_64 | 16 | TTGAACCTTGGGCCTGGCTGGCCGGTCCGCCTCACCGCGTGCACT GGTCCGGCCGGGCCTTTCCTTCTGGGGAGCCGCATGGCCTTCACT GGCCGTGTCGGGGAACCAGGACTTTTACTTTG |
| oligotroof;oligo-mesotroof_70 | 17 | TTGGATTTCTGGTTTGAGCGTCCGGTCGACCTTGTGTCTGTACTAT GGATGTTTGGGCCATATTTGTGAGGATGCCTTTCTGCCATTGAGT TGGTGGTTGGGTAGACTTGCATCGTTTACTGTG |
| uniqueoligo-mesotroof_117 | 18 | TTGGATCTCAGTTCTGGACGGGCGGTTCATGTAGCAATGATACCG TTCGTACCGGACCCATTGCTGGTTCTACCTAGGTGTCCTTCACTGG |
| | | GTGCCCTGGGAGACCAGAAAGTTTACTTTG |
| uniqueoligo-mesotroof_477 | 19 | TTGAACCTTGGGCCTGGCTGGCCGGTCCGCCTCACCGCGTGCACT GGTTCGGCCGGGCCTTTCCCTCTGGGGAGCCTCATGTCCTTCACTG GGTGTGATGGGGAACCAGGACTTTTACTGTG |
| uniqueoligo-mesotroof_109 | 20 | TTGGATTTCTGTGTTGAATGTCCGGTCGATTCCCTTTGGGAGTGTG TACTTGTGACATTTGATGCCTTTTTTGTGAGGATGTTTTTCTGTCAT TAAGTTGATGGTTGAATGGACTTGCATCGTTTACTGTG |
| uniqueoligo-mesotroof_83 | 21 | TTGGATTTCTGGTCGTGGTAATCGGTCCGCCCTTAACGGGGTGTG TACCAGGTTCCTGCGTCCATCCTCGAGGAGAACGTATCTGGCATT AAGTTGTCGGGTATGATATCCTCGTCATTTACTGTGA |
| uniqueoligo-mesotroof_330 | 22 | TTGAATTTCTGGCCGTGGGTTCCGGTCCGTCCCGCAAGGGTTGTG TACCAGGAATTCGCGGCCATCCTCGAGGAGAGTATATCTGGCATT CAGTTGTTGGGTATAGGACCCTCGTCATTTACTGTGA |
| oligo-mesotroof_81 | 23 | TTGGATCTCGGCTCTCAGGGGGCGGTCCGTTATTTGACGCGACTG CCCCATTTGAGAGTGTTTCTTCGGCGGAACTCCCGGGGTGCTCTTC GCCGAGTGTCCCGTTGGGGGCCGCCAGTTTTACTTTG |
| oligo-mesotroof_83 | 24 | TTGAATTTTGGACCTGGTTGGGCGGTCTTCTCATTGAGATGCACTG CTCTGACTGGGTCTTTCCTTCTGGCGAACTCTTGTGCTATTCATTTA GTGCGATTGGGAACCAGGACTTTTACTTTGA |
| oligo-mesotroof_85 | 25 | TTGAATTTTGGGCCTGGTTGGTTGGTCTGCCTTTGGTATGAACTGA CCGACTGGGCCTTTCCTTCTGGCGAACTATTGTGCTATTCATTTAG TGCGATAGGGAACCAGGACTTTTACTTTG |
| oligo-mesotroof_86 | 26 | TTGGATTTCTGGAAATAGCGAGTGAAATATCCTTTACGGAGATG TTGCTCGCATTATCCATCTTTGAAGGATAGAAGTCTGGCATTTGGT TGTCGGGCTACTGTGCTTCGTCATTTACTGTGA |
| oligo-mesotroof; mesotroof_87 | 27 | TTGAATTTCTGGTCTCGGACAGCGGTCTGCCTCAACCGAGGTATG TACTATCTGTCTAAGTCCATCCTCGGGGAAAACTTATCTCTGATTA AGTTCAGGGGTATGTGATCCTCGTCATTTACTGTGA |
| oligo-mesotroof; mesotroof_90 | 28 | TTGGATTTCTGGTAGCGACATACGGTCCGCCCCTCACGGGGTGCG CACTAGTATGTTGCATCCATCCTCGAGGAGAACGTGTCTGGCATT CAGTTGTCGGATACGGTATCCTCGTCATTTACTGTGA |
| oligo-mesotroof; mesotroof_96 | 29 | TTGAATTTCTGGTTTCGGAGTGCGGTCTGCCCCGTGGGGTAAGTA TCAGTGCTTCGGAGCCATCCTCGAGGAGAACATATCTGTGATTGA GTTCATGGGTATGGGAGCCTCGTCATTTACTGTGA |

**Table 3 Diatomeae 18S marker sequences for mesotrophic surface water**

| Class | SEQ ID NO | sequence |
|---|---|---|
| oligo-mesotroof; mesotroof_87 | 27 | TTGAATTTCTGGTCTCGGACAGCGGTCTGCCTCAACCGAGGTATG TACTATCTGTCTAAGTCCATCCTCGGGGAAAACTTATCTCTGATTA AGTTCAGGGGTATGTGATCCTCGTCATTTACTGTGA |
| oligo- | 28 | TTGGATTTCTGGTAGCGACATACGGTCCGCCCCTCACGGGGTGCG |
| mesotroof;mesot roof_90 | | CACTAGTATGTTGCATCCATCCTCGAGGAGAACGTGTCTGGCATT CAGTTGTCGGATACGGTATCCTCGTCATTTACTGTGA |
| oligo-mesotroof; mesotroof_96 | 29 | TTGAATTTCTGGTTTCGGAGTGCGGTCTGCCCCGTGGGGTAAGTA TCAGTGCTTCGGAGCCATCCTCGAGGAGAACATATCTGTGATTGA GTTCATGGGTATGGGAGCCTCGTCATTTACTGTGA |
| mesotroof_104 | 30 | TTGGATTTCGGATGGGAGTTGCCGGTCCGTCGTTACGATGTGCAC TGGCCGCTCCTATCTTGTTGTCGGGGACTAGCTCCTTGGATTCACT TCCTGGGACTAGGAGCTGACGAGGTTACTTTGA |
| mesotroof_107 | 31 | TTGGATTTTGGGCTGGAGGATTCGGTCTGCCTTACGGGTTGATAC TGTTTCCTCCATCCTTCTTGCCGGGGACGCGCTCTGGCCTTCACTG GCTGGGGCGCGGAGTCGGCGATGTTACTTTG |
| mesotroof_115 | 32 | TTGGATTTGTGGGTGGAGGTGCTGGCCCAACGCTTTGCGTGGGT GCTGGCTACCCTCTGTCCATCCTTGGGTGGAACCTGTGTGGCATTA AGTTGTCGTGCAGGGGATGCCCATCTTTTACTGTG |
| mesotroof_117 | 33 | TTGAATTTCTGGTCTCGGACAGCGGTCTGCCTCAACCGAGGTATG TACTATCTGTCTAAGTCCATCCTCGGGGAAAACTTATCTCTGATTC AGTTCAGGGGTATGTGATCCTCGTCATTTACTGTGA |
| mesotroof;meso-eutroof_123 | 34 | TTGGATTTCGGGTGGGTACCGCCGGTCTCCCATTGGGTATGTACT GGCGTGGCCCGCCTTGCTGTCGGGGACGATCTCCTGGGCTTCACT GTCCGGGAATCGGAGTCGACGAGGTTACTTTGA |
| mesotroof;meso-eutroof_131 | 35 | TTGGATTTGTGGATGGGCCGTCCGGTGCGCTCTTTGAGCGTTTC ATGGGTAGCTCGTCCATCCTTGGGTGGAATCTGTGTGGCATTAAG TTGTCGTGCAGGGGATGCCCATCTTTTACTGTG |
| mesotroof;meso-eutroof_136 | 36 | TTGGATCTCGGGTCCAGGCTTGCGGTTCGCCTCGCGGCGATTACT GCTCGTCCTGACCTACCTCCCGGTTTTCCCTTGGTGCTCTTCGCTG AGTGCTTCGGGTGGCCGGAACGTTTACCTTG |
| mesotroof;meso-eutroof_144 | 37 | TTGGACCTTGGGCCGGGTCGGCCGGTCCGCCTCACGGCGAGCAC CGACCTACTCGACTCTATTGCCGGCGATGCGCTCCTAGCCTTAATT GGCCGGGTCGTGCCTCCGGCGCCGTTACTTTGA |
| mesotroof;meso-eutroof_147 | 38 | TTGGATTTCGGGTGGGTACCGCCGGTCTCCCATTGGGTAGTACTG GCGTGGCCCGCCTTGCTGTCGGGGACGATCTCCTGGGCTTTACTG TCCGGGAATCGGAGTCGACGATGTTACTTTGA |
| mesotroof;meso-eutroof_148 | 39 | TTGGATTTGTGGATGGGCCGTCCGGTGCGCTCTTTGAGCGTTAC ATGGGTAGCTCGTCCATCCTTGGGTGGAATCTGTGTGGCATTAAG TTGTCGTGCAGGGGATGCCCATCTTTTACTGTG |
| mesotroof;meso-eutroof_150 | 40 | TTGGATTTGTGGATGGGCCGTCCGGTGTGCTCTTTTGAGCGTTAC TTGGGCAGCTCGTCCATCCTTGGGTGGAATCTGTGTGGCATTAAG TTGTCGTGCAGGGGATGCCCATCTTTTACTGTG |
| mesotroof;meso-eutroof_156 | 41 | TTGGATTTGTGGCAGTCGCATGCGGCCCGGCATTCGTGTCGGTGT TTGCTCGCGTCGCCATCCTTGGGTGGAACCTGTGTGGCATTAGGT TGTCGTGCAGGGGATGCCCATCATTTACTGTG |
| mesotroof;meso-eutroof_167 | 42 | TTGGACTTTAGGCAAGGCGGTCTGGTCGCCCACTGGGTGTACTGG GCTCGCCGCGCCTCACCTTCGGGGAACTAGTTGTGCTTCGGTGCG CTAGGGAACCGAGCCTTACTTTGA |

**Table 4 Diatomeae 18S marker sequences for meso-eutrophic surface water**

| Class | SEQ ID NO | sequence |
|---|---|---|
| mesotroof;meso-eutroof_123 | 34 | TTGGATTTCGGGTGGGTACCGCCGGTCTCCCATTGGGTATGTACT GGCGTGGCCCGCCTTGCTGTCGGGGACGATCTCCTGGGCTTCACT GTCCGGGAATCGGAGTCGACGAGGTTACTTTGA |
| mesotroof;meso-eutroof_131 | 35 | TTGGATTTGTGGATGGGCCGTCCGGTGCGCTCTTTGAGCGTTTC ATGGGTAGCTCGTCCATCCTTGGGTGGAATCTGTGTGGCATTAAG TTGTCGTGCAGGGGATGCCCATCTTTTACTGTG |
| mesotroof;meso-eutroof_136 | 36 | TTGGATCTCGGGTCCAGGCTTGCGGTTCGCCTCGCGGCGATTACT GCTCGTCCTGACCTACCTCCCGGTTTTCCCTTGGTGCTCTTCGCTG AGTGCTTCGGGTGGCCGGAACGTTTACCTTG |
| mesotroof;meso-eutroof_144 | 37 | TTGGACCTTGGGCCGGGTCGGCCGGTCCGCCTCACGGCGAGCAC CGACCTACTCGACTCTATTGCCGGCGATGCGCTCCTAGCCTTAATT GGCCGGGTCGTGCCTCCGGCGCCGTTACTTTGA |
| mesotroof;meso-eutroof_147 | 38 | TTGGATTTCGGGTGGGTACCGCCGGTCTCCCATTGGGTAGTACTG GCGTGGCCCGCCTTGCTGTCGGGGACGATCTCCTGGGCTTTACTG TCCGGGAATCGGAGTCGACGATGTTACTTTGA |
| mesotroof;meso-eutroof_148 | 39 | TTGGATTTGTGGATGGGCCGTCCGGTGCGCTCTTTGAGCGTTAC ATGGGTAGCTCGTCCATCCTTGGGTGGAATCTGTGTGGCATTAAG TTGTCGTGCAGGGGATGCCCATCTTTTACTGTG |
| mesotroof;meso-eutroof_150 | 40 | TTGGATTTGTGGATGGGCCGTCCGGTGTGCTCTTTTGAGCGTTAC TTGGGCAGCTCGTCCATCCTTGGGTGGAATCTGTGTGGCATTAAG TTGTCGTGCAGGGGATGCCCATCTTTTACTGTG |
| mesotroof;meso-eutroof_156 | 41 | TTGGATTTGTGGCAGTCGCATGCGGCCCGGCATTCGTGTCGGTGT TTGCTCGCGTCGCCATCCTTGGGTGGAACCTGTGTGGCATTAGGT TGTCGTGCAGGGGATGCCCATCATTTACTGTG |
| mesotroof;meso-eutroof_167 | 42 | TTGGACTTTAGGCAAGGCGGTCTGGTCGCCCACTGGGTGTACTGG GCTCGCCGCGCCTCACCTTCGGGGAACTAGTTGTGCTTCGGTGCG CTAGGGAACCGAGCCTTACTTTGA |
| uniquemeso-eutroof_189 | 43 | TCGACATATCTCGATACCAGACATCTTTCTGCACTTCTACCTCTGGT AGTTGTCGTAGAACCTTTACTTTGA |
| uniquemeso-eutroof_190 | 44 | TTGAACCTTGGGTTTGGTTGGTCGGTCCGCCTCACCGCGTGCACT GATCCGACCGAATCTTTCCTTCTGGGGAACCGTATGCCCTTCACTG GGTGTGCTGGGGAACCAGGACTTTTACTTTG |
| meso-eutroof_175 | 45 | TTGGATCTGAGGACTCGAATTGGTGGTGCAGCAAACGCTGTTACT GCTGATTCGGCCTACCTAGTCGGTGCTCGCTCTTGGTGTTCTTAAT TGAATGCCCTGAGTGCCCGACATGTTTACTTTGA |
| meso-eutroof_176 | 46 | TTGAACCTTGGGCCTGGCTGGCCGGTCTGCTTCACCGCATGTACT GGTCTGGCTGGGTCTTTCCTCTTGGGGAACCGTATGCCCTTTACTG GGTGTACTGGGGAACCAAGGCTTTTACTTTG |
| meso- | 47 | TTGGATCTCGGGTCCAGGCTTGCGGTTCGCCTCGCGGCGATTACT |
| eutroof_178 | | GCTCGTCCTGACCTACCTCCCGGTTTTCCCTTGGTGCTCTTCGTTGA GTGCTTCGGGTGGCCGGAACGTTTACCTTG |
| meso-eutroof_180 | 48 | TTGGATTTCTGTTCAGGACGACCGGTCCGCCCTCTGGGTGTGTATC TGGCTCGGCCTGGGCATCTTCTTGGAGAACGTCGCTGCACTTGAC TGTGTGGCACGGTATCCAGGACTTTTACTTTGA |
| meso-eutroof_182 | 49 | TTGGATTCCGGGAGTGAGCCGCCGGTCGCCGCCCGCGGTCGATA TCGGCGCTCTTCCTTACCAGCCTCGGCCGCCGCCTGTGCTTCGCTG CTCGGGCGCGCGCCGCGGCGATGTTACCTTGA |
| meso-eutroof_212 | 50 | TTGAACCTTGGGCCTGGCTGGTCGGTCCGCCTCACCGCGTGTACT GATCCGGCCGGGCCTTTCCTTCTGGCTAGCCGCATGCCCTTCACTG GGTGTGTTGGTGAACCAGGACTTTTACTTTG |
| meso-eutroof_218 | 51 | TTGGATTTCTGCTGAGGATGACTGGTCCGCCCTCTGGGTGAGTAT CTGGCTCAGCCTTGGCATCTTCTTGGAGAACACAGCTGCACTTCAC TGTGTGGTGTGGAATCCAGGACCTTTACTTTGA |
| meso-eutroof_220 | 52 | TTGGATCTCGGGTACTAGCATATGGTACGCTTATTGCTGTTACTAT ATGTCTGGTCCTAATTCATCGGTTGGCTGTTGATGTCCTTTATTGG GTGTTTTCAGTTGCCGATACGTTTACTTTGA |
| meso-eutroof_227 | 53 | TTGGATTTGTGGATGATCTTGGTGTCCACATTATTGTTGGTACTAT TGAGATTGTCCATCTTTGGGTGGAATCTGTGTGGCATTAGGTTGT CGTGCAGGGGATGCCCATCGTTTACTGTG |
| meso-eutroof_232 | 54 | TTGAACTTTGGGCCTGGGCGGGCGGTCTACCTATGGTTTGTACTG CTGCGGCCGGGTCTTTCCTTCTGGCTACCCCTCCGGGGGGAACCA GGACGATTACTTTGA |
| meso-eutroof_235 | 55 | TTGGATCTCGGGAACTAGCATGTGGTACGCTTTATTGCTGTTACTA CATGTCTGGTCCTAATCAATCGGTTGGCTGTTAATGCCCTTTACTG GGTGTTTTCAGTAGCCGGTACGTTTACTTTGA |
| meso-eutroof; eutroof_258 | 56 | TTGGATCTGCGCCTTCGGACTCGGTCCGCCCAACGGGTGTGAACT GGGATCCAAGGCTTATACTGCTGGTTTTCCCTTGATGCTCTTTACT GGGTGTCTTGGGTGGCTAGCGAGTTTACTTTG |
| meso-eutroof; eutroof_260 | 57 | TTGGATTTGTGGTGTGTGGTGTGTTCCGGGCACTTGTTGTCTGAG TAACACGCTGCTGCCATCCTTGGGTGGAACCTGTATGGCATTAGG TTGTCGTGCAGGGGATGCCCATCTTTTACTGTG |
| meso-eutroof; eutroof_296 | 58 | TTGGATTTTGGGTTGGGCCGACCGGTCCGCCTCTGGGTGTGCACC GGTTGTCTCGTCCCTTCTACCGGCGATGCGTTCCTGGCCTTAACTG GCCGGGTCGTGCCTCCGGTGCTGTTACTTTG |
| meso-eutroof; eutroof_331 | 59 | TTGAACCTTGGGCCTAGCCGGCCGGTCCGCCTCACCGCGTGCACT GGCTCGGTTGGGCCTTTCTTTCTGGAGAACCCCATGTCCTTCACTG GGTGTGGTGGGGAACCAGGACTTTTACCGTGA |
| meso-eutroof; eutroof_336 | 60 | TTGAATTTTGGGCCTGGTCGGGCGGTCTTGCTGCTTTATTGTGGTA AGCACTGCTTGGTCGGGTCTTTCCTTCTGGGGAACTAGTGCTGCCT TTTACTGGGACTGGCCTAGCGATCCAGGACTTTTACTTTGA |
| meso-eutroof; eutroof_352 | 61 | TTGAAGTTTGGTCTCGGACGCTGGGTCTGCTTAATTGCATGTACTT GACGGTCCGAGACTTCCTTCTTGGTGAACGGCCGCCTTCGGGTGG TCCGGAACCAGGACTATTACTTTG |
| meso-eutroof; eutroof_358 | 62 | TTGGATTTCTGGCAGGAGCGTCCGGTCTTGCACTCTGTGCGGAA CTTGGATGTCTCTGGCCATCTTTGGGGATATCCTGTTTGGCATTAA GTTGTCGGGCAGGGGATACCCATCGTTTACTGTG |
| meso-eutroof; eutroof_372 | 63 | TTGGATTTCGGGACGGAGACGTCGGTCCTCCCTTGTGGTCGATAC TGACTCTCTTCCTTAATTGCCTCGAGCGTCGCCTAGTCTTCATTGCC TGGGCGCGCTACGCGGCGCCGTTACCTTGA |
| meso-eutroof; eutroof_381 | 64 | TTGGACTTTGGGTTGGGTTGATCGGTCCGCCTTCTGGTGTGTACCT ATCGTCTCGTCCCTTCTGTCGGCGATACGTTCCTTGCCTTAATTGG CCGGGTCGTGCCTCCGACGCTGTTACTTTGA |
| meso-eutroof; eutroof_406 | 65 | TCGAATTTCGGGCAGGATGCTATGGTCCACCTCTTAGGTGGACC AATGTGTCTAGTCCTATTTCTAGGGACGCTATAGCCTTAACGGGT GTGTAGCCGTGTCTAGGACTTTTACTTTGA |
| meso-eutroof; eutroof_421 | 66 | TTGGATTTGTGGCGTTCGCGTGCGGCCCGGCATTATTGTCGGTGT TTGCTAGCGTCGCCATCCTTGGGTGGAACCAGTGTGGCATTAAGT TGTCGTGCTGGGGATGCCCATCGTTTACTGTG |
| meso-eutroof; eutroof_431 | 67 | TTGAACCTCGGGCCTGGCTGGCCGGTCCGCCTCACCGCGTGCACT GGTCCGGCCGGGTCTTTCCTCCTGTGGAACCTCGGGTCCTTCACTG GGCTCGGCGGGAAAACAGGACATTTACTTTG |
| meso-eutroof; eutroof_502 | 68 | TTGAACTTTAGGCAAGGCGGCCTGGTCACCCACTGGGTGTACTGG GCTCGCCGCGCCTCACCTTCGGGGAACTAGTCGTGCTTCGGTGCG CTAGGGAACCGAGCCTTACTTTGA |
| meso-eutroof; eutroof_518 | 69 | TTGGATTTCGGGTGGGTACCGCCGGTCTCCCTTTGGGTATGTACT GGCGTGGCCCGCCTTGCTGTCGGGGACGGTCTCCTGGGCTTCACT GTCCGGGAATCGGAGTCGACGAGGTTACTTTGA |

**Table 5 Diatomeae 18S marker sequences for eutrophic surface water**

| Class | SEQ ID NO | sequence |
|---|---|---|
| meso-eutroof;eutroof_258 | 56 | TTGGATCTGCGCCTTCGGACTCGGTCCGCCCAACGGGTGTGAA CTGGGATCCAAGGCTTATACTGCTGGTTTTCCCTTGATGCTCTT TACTGGGTGTCTTGGGTGGCTAGCGAGTTTACTTTG |
| meso-eutroof;eutroof_260 | 57 | TTGGATTTGTGGTGTGTGGTGTGTTCCGGGCACTTGTTGTCTG AGTAACACGCTGCTGCCATCCTTGGGTGGAACCTGTATGGCAT TAGGTTGTCGTGCAGGGGATGCCCATCTTTTACTGTG |
| meso-eutroof;eutroof_296 | 58 | TTGGATTTTGGGTTGGGCCGACCGGTCCGCCTCTGGGTGTGCA CCGGTTGTCTCGTCCCTTCTACCGGCGATGCGTTCCTGGCCTTA ACTGGCCGGGTCGTGCCTCCGGTGCTGTTACTTTG |
| meso-eutroof;eutroof_331 | 59 | TTGAACCTTGGGCCTAGCCGGCCGGTCCGCCTCACCGCGTGCA CTGGCTCGGTTGGGCCTTTCTTTCTGGAGAACCCCATGTCCTTC ACTGGGTGTGGTGGGGAACCAGGACTTTTACCGTGA |
| meso-eutroof;eutroof_336 | 60 | TTGAATTTTGGGCCTGGTCGGGCGGTCTTGCTGCTTTATTGTG GTAAGCACTGCTTGGTCGGGTCTTTCCTTCTGGGGAACTAGTG CTGCCTTTTACTGGGACTGGCCTAGCGATCCAGGACTTTTACTT TGA |
| meso- | 61 | TTGAAGTTTGGTCTCGGACGCTGGGTCTGCTTAATTGCATGTA |
| eutroof;eutroof_352 | | CTTGACGGTCCGAGACTTCCTTCTTGGTGAACGGCCGCCTTCG GGTGGTCCGGAACCAGGACTATTACTTTG |
| meso-eutroof;eutroof_358 | 62 | TTGGATTTCTGGCAGGAGCGTCCGGTCTTGCACTCTGTGCGGG AACTTGGATGTCTCTGGCCATCTTTGGGGATATCCTGTTTGGC ATTAAGTTGTCGGGCAGGGGATACCCATCGTTTACTGTG |
| meso-eutroof;eutroof_372 | 63 | TTGGATTTCGGGACGGAGACGTCGGTCCTCCCTTGTGGTCGAT ACTGACTCTCTTCCTTAATTGCCTCGAGCGTCGCCTAGTCTTCA TTGCCTGGGCGCGCTACGCGGCGCCGTTACCTTGA |
| meso-eutroof;eutroof_381 | 64 | TTGGACTTTGGGTTGGGTTGATCGGTCCGCCTTCTGGTGTGTA CCTATCGTCTCGTCCCTTCTGTCGGCGATACGTTCCTTGCCTTA ATTGGCCGGGTCGTGCCTCCGACGCTGTTACTTTGA |
| meso-eutroof;eutroof_406 | 65 | TCGAATTTCGGGCAGGATGCTATGGTCCACCTCTTAGGTGGGA CCAATGTGTCTAGTCCTATTTCTAGGGACGCTATAGCCTTAAC GGGTGTGTAGCCGTGTCTAGGACTTTTACTTTGA |
| meso-eutroof;eutroof_421 | 66 | TTGGATTTGTGGCGTTCGCGTGCGGCCCGGCATTATTGTCGGT GTTTGCTAGCGTCGCCATCCTTGGGTGGAACCAGTGTGGCATT AAGTTGTCGTGCTGGGGATGCCCATCGTTTACTGTG |
| meso-eutroof;eutroof_431 | 67 | TTGAACCTCGGGCCTGGCTGGCCGGTCCGCCTCACCGCGTGCA CTGGTCCGGCCGGGTCTTTCCTCCTGTGGAACCTCGGGTCCTT CACTGGGCTCGGCGGGAAAACAGGACATTTACTTTG |
| meso-eutroof;eutroof_502 | 68 | TTGAACTTTAGGCAAGGCGGCCTGGTCACCCACTGGGTGTACT GGGCTCGCCGCGCCTCACCTTCGGGGAACTAGTCGTGCTTCG GTGCGCTAGGGAACCGAGCCTTACTTTGA |
| meso-eutroof;eutroof_518 | 69 | TTGGATTTCGGGTGGGTACCGCCGGTCTCCCTTTGGGTATGTA CTGGCGTGGCCCGCCTTGCTGTCGGGGACGGTCTCCTGGGCTT CACTGTCCGGGAATCGGAGTCGACGAGGTTACTTTGA |
| uniqueeutroof_355 | 70 | TTGGATTTCTGACAGGGGCGCATGGTGGGCTCGCAAGGGTC CCGACCGATGTCAGTCTTTGTCATCCTTGGGGAGAGCCGTATT GCCATTAACTTGGTAGTGCGGGGATCCTCATCGTTTACTGTGA |
| uniqueeutroof_269 | 71 | TTGACCATTTGGCCTGGCTGGCAGGTCCGCCTCACCGCGTGCA CTTGTCCGGCCGGGCCTTTCTTTCTGGAGAACCGCATGCCCTTC ACTGGGCGTGTTGGGGACCAGGACTTTTACTTTG |
| uniqueeutroof_357 | 72 | TTGAACCTTGGGCCTGGCTGGCTGGTCCGCCTCACCGCGTGTA CTGGTTGGCCGGGCCTTTCCTTCTGGAGAACCCCATGCCCTTC ACTGGGTGTGCGGGGGAACCAGGACTTTTACTGTG |
| uniqueeutroof_187 | 73 | TTGGACTTCTGGCAGGAGCGTCTGGTCTCACACTCTGTGCGGG AACTTGGATGTCTCTGGCCATCTTTGGGGATATCCTGTTTGGC ATTAAGTTGTCGGGCAGGGGATACCCATCGTTTACTGTG |
| uniqueeutroof_289 | 74 | TTGACCATTTGGCCTGGCTGGCGGGTCCGCCTCACCGCGTGCA CTTGTCCGGCCGGGCCTTTCTTTCTGGAGAACCGCATGCCCTTC ACTGGGCGTGTTGGGGACCAGGACTTTTACTTTG |
| eutroof_527 | 75 | TTGGATTTGTGGTTGCTCGCTGGGTCCTCTTTTTGAGGCATCCG GTTGGGTGACCATCCTTGGGTGGATGTTGCGTGGCATTCGGTT GTCGCGCAGGGGATGCCCATCGTTTACTGTG |
| eutroof_529 | 76 | TTGGACCTTGGGCCGGGTAGGTCGGTCCGCCTCTCGGGTGTG AACCAGCCTCCTCGTCCCTTCTGCCGGCGATGCGCTCCTGTCCT TAATTGGCCGGGTCGTGCCTCCGGCGCTGTTACTTTGA |
| eutroof_535 | 77 | TTGGATTTCGGAGCGGGCCCGTCGGTCCACCGCAAGGTGAGC TACTGGCGGGTCTGTTCTTCTTCGCAAAGACTGCAGGTGCACT TCGCTGTGTGCTTGTCGGATTTGTGACGTTTACTTTG |
| eutroof_542 | 78 | TTGGATTTCTGGATACGGCAGCCGGTCCTCCCTTAACGGGGTT GTGTACTAAGTTGCTGTGTCCATCCTCGAGGAGAACATGCCTG GCATTTATTTGTCGGGTATGGTATCCTCGTCATTTACTGTG |
| eutroof_543 | 79 | TTGAATTTCTGGTCCCGGAACACGGTCAGCTCCAAGGAGTTCG TACCTGTGGTCTAGGACCATCCTCGGGGAGAACATATCTGCCA TTAAGTTGGTGGGTATGGGACCCTCGTCATTTACTGTGA |
| eutroof_544 | 80 | TTGGATTTCGGGTGGGTTCTAGCGGTCCGCCTCTGGTGTGTAC TGCTATGGCTCACCTTTCTGTCGGGGACCAGCTCTTGGGCTTA ACTGTCCGGGACTGGGAATCGGCGAGGTCACCGTGA |
| eutroof_548 | 81 | TTGGACTTGTGCATGTCCCTGTATGTCCATATTGTTTGGTGCCT TCAAGGGCGTGCATCCTTGGGTGAAACCTGTGTGGCATTAGG TTGTCGTGTAGGGAATGCTCATCGTTTACTGTG |
| eutroof_572 | 82 | TTGGATTTCGGGTGGGGCCTGCCGGTCCGCCGTTTCGGTGTGC ACTGGCAGGGCCCACCTTGTTGCCGGGGACGGCTCCTGGGC TTCACTGTCCGGGACTCGGAGTCGGCGCTGTTACTTTGA |
| eutroof_577 | 83 | TTGAATTTCTGGTCTCGGACATCGGTCTGCCTCGAACGAGGTA CGTACCAGTTGTCTGAATCCATCCTCGGGGAAAACGTGTCTGT CATTCAGTTAATGGGCACGGGATCCTCGTCATTTACTGTGA |
| eutroof_589 | 84 | TTGGACTTTGGGTTTGGCCGGCCGGTCCGCCCTCGTGGTGTGC ACCGGTCGTCTCATCCCTTCTACCGGCGATACGCTCCTGTCCTT AATTGGCCGGGTCGTGCCTCCGGTGCTGTTACTTTGA |
| eutroof_600 | 85 | TTGAAATTGGGGAATTATTTCGATTGGAGACTTATGGTCGACA ATTGATTTATCCTTCGTCGTCATGTATATTGGGAAGGTTCTTAA TTGGATTTTCTTGATAACTGACAAGTTTACTTTG |
| eutroof_612 | 86 | TTGGATTTCGGGCGGGGCCTGCCGGTCCGCCTTTGGTGTGCAC TGGCAGGGTCCGCCTTGCTGTCGGGGACGGTTCCTGGGCTT TACTGTCTGGGACTCGGAGTCGACGTGGTTACTTTGA |
| eutroof_640 | 87 | TTGGATCTCGGCACCCCTCTGCCGGTCGCCTGTTCGGGCGGAC TGGCACGATCGGGGTGTGACGCTTTGGCGGAGGCCCGGGGG TGCTCTTGACCGAGTGTCCCCGCGGTGCCGTCAGGTTTACTTT G |
| eutroof_654 | 88 | TTGGATTTCTGGTAGGATAACTTTGCCCTCTTGATGAGTGCGC AGAGTGATCCTGCCATCCTTCTGTTAACCCTTCAGGCATTCTTT TGTTTGTTGGGGGCTCAGATATTTTACCTTGA |
| eutroof_659 | 89 | TTGGATTTCTGGCAGGAGTGACCGGCCATGCACTCTGTGCGTG AGCTGTGTCATTCTGGCCATCCTTGGTGAGATCCTGTTTGGCA TTAAGTTGTCGGGCAGGGGATAACCATCGTTTACTGTGA |
| eutroof_665 | 90 | TTGGATTTCTGTTTTGAACGTCCGGTCCGCTTCTTAGAAGTGTG TTACTAGGATGTTTGAGACATTTTTTGTGAGGATGTTCTTCTGC CATTCAGTTGGTGGTTGAATAGACTTGCATCGTTTACTGTG |
| eutroof_672 | 91 | TTGGAAATTGGGCACGGCCGGGCGGTCCACCTTAGGGTGCGT ACTGCTCAGTGGCGTTTCGGCGTCACGAGGCTGAGCCTTGGC CGCGTCCTCGTGCAGGGGCAACCTTGTACGCGGGACAGGCAG TTACCTTGA |
| eutroof_688 | 92 | TTGGATTTTGGGTTGGGCCGACCGGTCCGCCTCTGGGTGTGCA CCGGTTGTCTCGTCCCTTTTACCGGCGATGCGTTCCTGGCCTTA ACTGGCCGGGTCGTGCCTCCGGTGCTGTTACTTTGA |
| eutroof; hypereutroof_750 | 93 | TTGAATTTCTGGTCTCGGAACACGGTCAGCTCCTAGGAGTTCG TACCTGTGAGCCGGGGCCATCCTCGGGGAGAACATATCTGCC ATTAAGTTGGTGGGTATGGGACCCTCGTCATTTACTGTGA |
| eutroof; hypereutroof_763 | 94 | TTGGATTTCTGCCGAGGACGACCGGTCCGCCCTCTGGGTGAG CATCTGGCTCGGCCTGGGCATCTTCTTGGAGAACGTATCTGCA CTTGACTGTGTGGTGCGGTATTCAGGACTTTTACTTTGA |
| eutroof; hypereutroof_787 | 95 | TTGAATTTTGGGCCTAGCTGGGTGGTCTAGCTTAACGGTTAGT ACTACTTTGGCTGGGTCTTTCCTTCTGGCTAACCAGTGTGTCCT TAACTGGGTGCGCTGGGGCTCCAGGACTTTTACTGTG |
| eutroof; hypereutroof_793 | 96 | TTGAACCTTGGGCCTGGCTGGCAGGTCCGCCTCACCGCGTGTA CTTGTCCGGCCGGGTCTTCCTTCTGGAGAACCTCATGCCCTTCA CTGGGCGTGTTGGGGACCAGGACTTTTACTTTG |
| hypereutroof_817 | 97 | TTGGATTTCTGGTAGGAATATCGATGTCAGATTGTTCTGTGCA GACATATCTTACCATCCTTCTGTTAACTTGGTTTGCTCTTCAAA GGCGGCCAAGGGCTCAGATATTTTACCTTGA |

**Table 6 Diatomeae 18S marker sequences for hypereutrophic surface water**

| Class | SEQ ID NO | sequence |
|---|---|---|
| eutroof; hypereutroof_750 | 93 | TTGAATTTCTGGTCTCGGAACACGGTCAGCTCCTAGGAGTTCG TACCTGTGAGCCGGGGCCATCCTCGGGGAGAACATATCTGCC ATTAAGTTGGTGGGTATGGGACCCTCGTCATTTACTGTGA |
| eutroof; hypereutroof_763 | 94 | TTGGATTTCTGCCGAGGACGACCGGTCCGCCCTCTGGGTGAG CATCTGGCTCGGCCTGGGCATCTTCTTGGAGAACGTATCTGCA CTTGACTGTGTGGTGCGGTATTCAGGACTTTTACTTTGA |
| eutroof; hypereutroof_787 | 95 | TTGAATTTTGGGCCTAGCTGGGTGGTCTAGCTTAACGGTTAGT ACTACTTTGGCTGGGTCTTTCCTTCTGGCTAACCAGTGTGTCCT TAACTGGGTGCGCTGGGGCTCCAGGACTTTTACTGTG |
| eutroof; hypereutroof_793 | 96 | TTGAACCTTGGGCCTGGCTGGCAGGTCCGCCTCACCGCGTGTA CTTGTCCGGCCGGGTCTTCCTTCTGGAGAACCTCATGCCCTTCA CTGGGCGTGTTGGGGACCAGGACTTTTACTTTG |
| Hypereutroof_817 | 97 | TTGGATTTCTGGTAGGAATATCGATGTCAGATTGTTCTGTGCA GACATATCTTACCATCCTTCTGTTAACTTGGTTTGCTCTTCAAA GGCGGCCAAGGGCTCAGATATTTTACCTTGA |

### Examples

### Example 1: DNA isolation from fresh water samples and sequencing

### DNA isolation from biological samples

The method consists of concentrating biological material through sedimenting by careful centrifugation for collecting eukaryotic material (18s rDNA fraction) followed by high speed centrifugation for collecting bacterial material (16s rDNA fraction).

Eukaryotic material is collected by overnight sedimentation of water samples of half a liter volume. Liquid is removed by pipetting until 50 ml is left with the sediment. This material is then centrifuged for 15 min at 300 g. The supernatant is removed by pipetting until 1 ml is left. The remaining sediment is considered the 18s rDNA fraction.

DNA is isolated from these samples with the following protocol:
1. In a 2 ml screw cap eppendorf tube the following components are mixed:
   a. 400µl 18s rDNA fraction
   b. 400µl 0.5 mm zirconium beads (Biospec.), washed in lysisbuffer (Agowa mag Mini DNA isolation kit art. nr. 40401)
   c. 500µl Phenol, water saturated with 10 mM Tris pH8.0.
   d. 300µl lysisbuffer (Agowa).
2. 2 minutes beadbeating, cooling down on ice
3. 10 minutes centrifugation at 10000 rpm
4. Transfer 500 µl supernatant to new 2 ml eppendorf tube filled with 1 ml binding buffer and 20 µl magnetic beads (Agowa mag Mini DNA isolation kit art. nr. 40401). Mix by pipetting.
5. Incubate 10 minutes at rT while shaking (1000 rpm).
6. Place tubes in magnetic separator, incubate 1 minute
7. Remove supernatant followed by removing magnet.
8. Add 400µl washing buffer 1, mix by pipetting
9. Incubate 5 minutes at rT while shaking (1000 rpm)
10. Place tubes in magnetic separator, incubate 1 minute
11. Remove supernatant followed by removing magnet
12.Add 200µl washing buffer 2, mix by pipetting
13. Incubate 5 minutes at rT while shaking (1000 rpm)
14. Place tubes in magnetic separator, incubate 1 minute
15. Remove supernatant followed by removing magnet
16. Dry magnetic beads at 55°C
17.Resuspend magnetic beads in 63µl elution buffer
18. Incubate 10 minutes at 55°C
19. Place tubes in magnetic separator, incubate 1 minute
20. Remove supernatant and bring to new cup.

This DNA is now ready for PCR amplification

### PCR amplification

The amplification mix contained 1 unit of Phusion Hot Start II High-Fidelity DNA Polymerase (Thermo Scientific, Pittsburgh, PA, USA) and 1x Phusion HF buffer (Thermo Scientific), 200 µM dNTP and 1 µM of each primer (18s F 5'-TGCAGTTAAAAAGCTCGTAG-3' and 18s R 5'-TAATCATCTTCGATCCCCTA-3'). After denaturation (98°C; 30 sec), 35 cycles were performed that consisted of denaturation (98°C; 10 sec), annealing (50°C; 30 sec), and extension (72°C; 30 sec)followed by a final extension step (72°C; 5 min). As a next step PCR products were analyzed with the Caliper Labchip GX (Perkin Elmer, Santa Clara, CA, USA) for exact sizing and quantitation. Based on the results of the Caliper Labchip GX analysis, the amplicons of the individual samples were pooled in equimolar amounts in one library. This pool was gel purified and amplicons of the expected size were eluted by means of the QIAquick PCR Purification Kit (Qiagen, Hilden, Germany) followed by AMpure bead purification (Beckman Coulter, Brea, CA, USA). This purified amplicon pool was then used for 454 pyrosequencing.

Sequencing data were processed as follows: data were trimmed by removing primer sequences and low-quality data, overlapping paired-ends were stitched together, sequences that did not have an exact match to the barcode, sequences that had an ambiguous base call (N) in the sequence. Sequences were grouped according to the barcode. We then aligned the sequences with the MOTHUR align function (Schloss et al., 2009. Appl Environ Microbiol 75: 7537-7541) using kmer search. Chimera.uchime (available in MOTHUR) was used to remove sequence chimera's from the dataset.

Unique sequences were aligned using the "align.seqs" command and the MOTHUR-compatible Eukaryotic SILVA SEED database. Sequences were taxonomically classified by the RDP-II Naive Bayesian Classifier using a 60% confidence threshold. Sequences were assigned to taxa according to the RDP training set using kmer search. In cases where reads were equidistant to multiple 18S reference sequences, and/or where identical 18S sequences were derived from longer sequences mapping to different taxa, reads were assigned to the lowest common taxon of at least 60% of the sequences.

### Example 2 Classification of fresh water samples

On basis of a hybridization assay with the 97 sequences from Tables 1 - 6 a number of samples of known trophic state was classified.

The following results were obtained:

**Table 7 Results of classification of 100 samples on basis of a hybridization assay with all 97 probes of Tables 1-6**

| | oligo | oligo-meso | meso | meso-eu | eutrophic | hyper | % |
|---|---|---|---|---|---|---|---|
| Assigned | 23 | 13 | 6 | 26 | 38 | 3 | 70 |
| 2 groups | 0 | 3 | 5 | 9 | 6 | 3 | 17 |
| Unassigned | 1 | 3 | 3 | 6 | 5 | 2 | 13 |

In this Table it shows that at least 70% of the samples are classified in the correct class when a six-class system was used. A further relatively large number of samples (17 out of 100) was assigned to two neighbouring classes, most probably because the sample was somewhat ambiguous because of the gradual changes from class to class. When looking at 5 classes (i.e. the classes eutrophic and hypereutrophic combined) the percentages for this joint class were: assigned 45 of 57 and unassigned 7/57. The remaining samples again would be classified as belonging to two groups.

## Claims

1. A method for developing a classifier for classifying the trophic state of surface water comprising the steps of:
a. taking samples of surface water of different trophic states;
b. determining the nucleotide sequence of 18S RNA of micro-organisms in said sample;
c. selecting for those sequences that are unique for a specific trophic state.

2. A method for classifying the trophic state of surface water comprising the steps of:
a. taking a surface water sample;
b. extracting the nucleotide sequence of the 18S RNA of the microorganisms in said sample;
c. checking for the presence of one or more of the nucleotide sequences depicted in any of Tables 1 - 6 in the extracted nucleotide sequences of step b;
d. classifying the sample on basis of the presence or absence of said sequences.

3. A method according to claim 2, wherein the classification is provided into three classes: eutrophic, mesotrophic and oligotrophic.

4. A method according to claim 2, wherein the classification is provided into 5 classes: eutrophic, meso-eutrophic, mesotrophic, oligo-mesotrophic and oligotrophic, specifically wherein the class of eutrophic is subdivided into two classes eutrophic and hypereutrophic.

5. A method according to any of claims 1 - 4, wherein the micro-organisms are diatomaceous micro-organisms.

6. A method according to any of claims 2 - 5, wherein the presence of one or more of the sequences of Table 1 is indicative for the oligotrophic state of the surface water.

7. A method according to any of claims 2 - 5, wherein the presence of one or more of the sequences of Table 3 is indicative for the mesotrophic state of the surface water.

8. A method according to any of claims 2 - 5, wherein the presence of one or more of the sequences of Table 5 is indicative for the eutrophic state of the surface water.

9. A method according to any of claims 4 - 5, wherein the presence of one or more of the sequences of Table 2 is indicative for the oligo-mesotrophic state of the surface water.

10. A method according to any of claims 4 - 5, wherein the presence of one or more of the sequences of Table 4 is indicative for the meso-eutrophic state of the surface water.

11. A method according to any of claims 4 - 5, wherein the presence of one or more of the sequences of Table 6 is indicative for the hypereutrophic state of the surface water.

12. A method according to any of claims 2 - 5, wherein said sequences of any of Tables 1- 6 are available on an array for hybridization with the sequences of the sample.

13. A method according to claim 12, where the array comprises from 50 to all of the sequences of Tables 1- 6, preferably wherein the array comprises more than 70, preferably more than 80, preferably more than 90 sequences.

14. Kit for the classification of the trophic state of surface water, comprising an array for hybridization, wherein said array is provided with at least 30 spots, each spot containing molecules comprising one of the sequences as depicted in any of Tables 1- 6.
